# EUROPEAN PATENT APPLICATION

(11) **EP 4 817 432 A1**
(43) Date of publication of application: **07.10.2026**
(21) Application number: 24897697.9
(22) Date of filing: 29.11.2024
(51) Int. Cl.: A23L 33/125, A23L 33/135, A61K 31/702, A61K 31/7016, A61P 1/04, A61P 25/16, A61P 29/00, C12P 19/04

(54) **COMPOSITION FOR CONTROLLING GROWTH OF FUSICATENIBACTER BACTERIA**

(30) Priority: 01.12.2023 JP 2023204246
(71) Applicant: Meiji Co., Ltd., Chuo-ku Tokyo 104-8306 (JP)
(72) Inventor: ISHIDA, Junya, Hachioji-shi, Tokyo 192-0919 (JP); ICHIKAWA, Ayumi, Hachioji-shi, Tokyo 192-0919 (JP); NEGISHI, Hiroki, Hachioji-shi, Tokyo 192-0919 (JP)
(74) Representative: TBK
(86) International application number: PCT/JP2024/042333
(87) International publication number: WO 2025/116015

(57) **Abstract**

An object of the present invention is to provide a means for controlling proliferation of Fusicatenibacter bacteria. Provided is a composition for controlling proliferation of Fusicatenibacter bacteria, containing any one selected from kojibiose and an oligosaccharide containing kojibiose as a constituent saccharide. In an aspect, the composition contains galactosyl kojibiose as any one selected from kojibiose and an oligosaccharide containing kojibiose as a constituent saccharide.

## Description

### TECHNICAL FIELD

The present invention relates to a composition for controlling proliferation of Fusicatenibacter bacteria.

### BACKGROUND ART

Fusicatenibacter bacteria have been discovered from intestinal bacterial flora of humans. Non Patent Document 1 has reported that the number of Fusicatenibacter saccharivorans is significantly smaller in a patient suffering from ulcerative colitis than that in a healthy person. In addition, Non Patent Document 1 suggests a mechanism in which inflammation is suppressed by administration of heat-killed bacterial bodies of Fusicatenibacter saccharivorans to oxazolone-induced colitis model mice and which is performed via suppression of IL-10 and IL-13 whose production is increased by oxazolone. Non Patent Document 2 has reported that symptom progression of Parkinson's disease is predicted in a patient group having a small number of bacteria belonging to the genus Fusicatenibacter, and such a decrease in the number of bacteria is not a result of the symptom progression. Furthermore, Non Patent Document 3 has reported that Fusicatenibacter bacteria are distributed with high frequency and number of bacteria in an adult, whereas a distribution frequency is low in an elderly person, and the number of bacteria in an elderly person is about 1/10 of that in an adult.

Regarding Fusicatenibacter bacteria, Patent Document 1 describes a composition for promoting proliferation of Fusicatenibacter bacteria, containing indigestible dextrin as an active ingredient. Patent Document 2 describes a composition containing a living bacterial strain belonging to the genus Fusicatenibacter for use in medical treatment, and describes that this composition is to be used in a method for treating or preventing a disease or a disorder selected from the group consisting of a disease, a disorder, or a condition of the central nervous system, a disorder of intestinal barrier function, a disease associated with increased intestinal permeability, an inflammatory disease, a metabolic disease, and a cardiovascular disease.

On the other hand, kojibiose is known to have an effect of promoting proliferation of bifidobacteria (for example, Non Patent Document 4). Furthermore, Patent Document 3 describes an in vivo lipid modulator containing, as an active ingredient, one or more selected from kojibiose, kojitriose, kojibiosyl glucoside, kojitetraose, and kojitriosyl glucoside. Patent Document 4 describes an administration dosage form containing a composition containing a microbiome control factor, exemplifies a sugar or a sugar alcohol as one of components that can be contained in the agent, and describes kojibiose as an example thereof. The agent is also described to be used for treating a subject with enterotoxemia, and inflammatory bowel disease is exemplified as the enterotoxemia. Furthermore, Patent Document 5 describes a method for treating a subject with a disease or a disorder associated with an undesired level of a metabolite, the method optionally including: a step of selecting a glycan polymer preparation based on modulation of production or a level of the metabolite by the glycan polymer preparation; and a step of administering the glycan polymer preparation in an amount effective for modulating the level of the metabolite, thereby treating the disease or disorder, and describes Crohn's disease, Parkinson's disease, and ulcerative colitis as examples of the disease. Patent Document 5 also describes that the glycan polymer contains an alpha-1,2 glycosidic bond, and describes kojibiose as an example of a glycan subunit starting material.

### PRIOR ART REFERENCES

### Patent Documents

Patent Document 1: JP 2020-83830 A (JP 7164869 B2)
Patent Document 2: WO 2021/098991 A (JP 2023-503410 A)
Patent Document 3: JP 2005-281188 A (JP 4746281 B2)
Patent Document 4: WO 2016/172658 A (JP 2018-513196 A)
Patent Document 5: WO 2018/106845 A (JP 2020-513410 A)

### Non Patent Documents

Non Patent Document 1: Inflamm Bowel Dis. 2016 Dec;22(12):2802-2810.
Non Patent Document 2: NPJ Parkinsons Dis. 2022 Jun 1;8(1):65.
Non Patent Document 3: PLoS One. 2015 May 22;10(5):e0126226.
Non Patent Document 4: Journal of agricultural and food chemistry. 2005, 53, 5192-5199

### SUMMARY OF THE INVENTION

### Object to be Achieved by the Invention

Since intestinal bacteria have been reported to be closely associated with health, there is a possibility that risk of various diseases can be reduced by controlling proliferation of important bacteria. In particular, it is considered that suppressing a decrease in Fusicatenibacter bacteria associated with aging by ingestion of prebiotics is effective in terms of reducing a risk of disease.

By controlling proliferation of Fusicatenibacter bacteria in the intestine by prebiotics, prevention and improvement of various diseases can be expected.

### Means for Achieving the Object

The present invention provides the following items.
[1] A composition for controlling proliferation of Fusicatenibacter bacteria, containing any one selected from kojibiose and an oligosaccharide containing kojibiose as a constituent saccharide.
[2] The composition according to the item 1, which is for use as a prebiotic or a synbiotic.
[3] A composition for treatment of any one selected from inflammatory bowel disease and Parkinson's disease, the composition containing any one selected from kojibiose and an oligosaccharide containing kojibiose as a constituent saccharide.
[4] The composition according to the item 3, wherein the treatment is performed via control of proliferation of Fusicatenibacter bacteria in the intestine.
[5] A composition for treatment of a disease or a condition improved by controlling proliferation of Fusicatenibacter bacteria in the intestine, the composition containing kojibiose and an oligosaccharide containing kojibiose as a constituent saccharide.
[6] The composition according to any one of the items 1 to 5, wherein the composition is obtained by allowing glucansucrase to act on a raw material composition containing lactose and sucrose.
[7] The composition according to any one of the items 1, 2, and 4 to 6, wherein the proliferation control is proliferation promotion.
[8] The composition according to any one of the items 1 to 7, containing galactosyl kojibiose as any one selected from kojibiose and an oligosaccharide containing kojibiose as a constituent saccharide.
[9] A food information providing method including:
   a step of acquiring information on intestinal bacterial flora of a subject;
   a step of deriving food information to be provided to the subject based on the information on the intestinal bacterial flora; and
   a step of providing the derived food information to the subject, wherein
   in the step of acquiring information, information on presence or absence or the amount of Fusicatenibacter bacteria is acquired from the information on the intestinal bacterial flora, and in the step of deriving food information, information on a food which is a composition containing any one selected from the group consisting of kojibiose and an oligosaccharide containing kojibiose as a constituent saccharide is derived based on the information on presence or absence or the amount of Fusicatenibacter bacteria.
[10] A method for controlling Fusicatenibacter bacteria in the intestine of a subject or a method for supporting a dietary life or health of the subject, the method including:
   a step of performing the method according to the item 9; and
   a step of providing a food to the subject based on the derived food information.
[11] The method according to the item 9 or 10, wherein the food is for use as a prebiotic or a synbiotic.
[12] The method according to any one of the items 9 to 11, further including a step of indicating the food information to be provided on a terminal of the subject.
[13] Use of any one selected from kojibiose and an oligosaccharide containing kojibiose as a constituent saccharide, for control of proliferation of Fusicatenibacter bacteria in intestinal bacterial flora.

[21] A composition containing any one selected from kojibiose and an oligosaccharide containing kojibiose as a constituent saccharide, for use in a method for controlling proliferation of Fusicatenibacter bacteria. Use of any one selected from the group consisting of kojibiose and an oligosaccharide containing kojibiose as a constituent saccharide in manufacture of a composition for controlling proliferation of Fusicatenibacter bacteria. A method or a non-therapeutic method for controlling proliferation of Fusicatenibacter bacteria, the method including a step of administering a composition containing any one selected from the group consisting of kojibiose and an oligosaccharide containing kojibiose as a constituent saccharide to a subject. Use or non-therapeutic use of a composition containing any one selected from the group consisting of kojibiose and an oligosaccharide containing kojibiose as a constituent saccharide, for controlling proliferation of Fusicatenibacter bacteria.
[22] The composition, the use in manufacture, the method or non-therapeutic method, or the use or non-therapeutic use according to the item 21, wherein the composition is for use as a prebiotic or a synbiotic.
[23] A composition containing any one selected from kojibiose and an oligosaccharide containing kojibiose as a constituent saccharide, for use in a method for treating any one selected from inflammatory bowel disease and Parkinson's disease. Use of any one selected from the group consisting of kojibiose and an oligosaccharide containing kojibiose as a constituent saccharide in manufacture of a composition for treating any one selected from inflammatory bowel disease and Parkinson's disease. A method or a non-therapeutic method for treating any one selected from inflammatory bowel disease and Parkinson's disease, the method or non-therapeutic method including a step of administering a composition containing any one selected from the group consisting of kojibiose and an oligosaccharide containing kojibiose as a constituent saccharide to a subject. Use or non-therapeutic use of a composition containing any one selected from the group consisting of kojibiose and an oligosaccharide containing kojibiose as a constituent saccharide, for treating any one selected from inflammatory bowel disease and Parkinson's disease.
[24] The composition, the use in manufacture, the method or non-therapeutic method, or the use or non-therapeutic use according to the item 23, wherein the treatment is performed via control of proliferation of Fusicatenibacter bacteria.
[25] A composition containing any one selected from the group consisting of kojibiose and an oligosaccharide containing kojibiose as a constituent saccharide, for use in a method for treating a disease or a condition improved by controlling proliferation of Fusicatenibacter bacteria in the intestine. Use of any one selected from the group consisting of kojibiose and an oligosaccharide containing kojibiose as a constituent saccharide in manufacture of a composition for treating a disease or a condition improved by controlling proliferation of Fusicatenibacter bacteria in the intestine. A method or a non-therapeutic method for treating a disease or a condition improved by controlling proliferation of Fusicatenibacter bacteria in the intestine, the method or non-therapeutic method including a step of administering a composition containing any one selected from the group consisting of kojibiose and an oligosaccharide containing kojibiose as a constituent saccharide to a subject. Use or non-therapeutic use of a composition containing any one selected from the group consisting of kojibiose and an oligosaccharide containing kojibiose as a constituent saccharide, for treating a disease or a condition improved by controlling proliferation of Fusicatenibacter bacteria in the intestine.
[26] The composition, the use in manufacture, the method or non-therapeutic method, or the use or non-therapeutic use according to any one of the items 21 to 25, wherein the composition is obtained by allowing glucansucrase to act on a raw material composition containing lactose and sucrose.
[27] The composition, the use in manufacture, the method or non-therapeutic method, or the use or non-therapeutic use according to any one of the items 21, 22, and 24 to 26, wherein the proliferation control is proliferation promotion.
[28] The composition, the use in manufacture, the method or non-therapeutic method, or the use or non-therapeutic use according to any one of the items 21 to 27, wherein the composition contains galactosyl kojibiose as any one selected from kojibiose and an oligosaccharide containing kojibiose as a constituent saccharide.
[29] A method for controlling proliferation of Fusicatenibacter bacteria in intestinal bacterial flora, the method including a step of causing any one selected from kojibiose and an oligosaccharide containing kojibiose as a constituent saccharide to be contained in the intestinal bacterial flora containing Fusicatenibacter bacteria.

### Effects of the Invention

By using any one selected from kojibiose and an oligosaccharide containing kojibiose as a constituent saccharide as a prebiotic, it is possible to increase the number of Fusicatenibacter bacteria in the intestine. Furthermore, by increasing the number of Fusicatenibacter bacteria in the intestine, prevention and improvement of various diseases can be expected.

### BRIEF DESCRIPTION OF THE DRAWINGS

Fig. 1 illustrates a change in abundance of Fusicatenibacter bacteria by galactosyl kojibiose (trisaccharide fraction) in a human fecal culture system in an amplicon sequence of a V3-V4 region by MiSeq (Illumina). A black circle indicates a transition of each subject, and a horizontal bar indicates an average value.
Fig. 2 illustrates a chromatogram of an enzymatic reaction liquid, and chromatograms of a trisaccharide fraction and a tetrasaccharide fraction after fractionation purification.

### MODES FOR CARRYING OUT THE INVENTION

The present embodiment relates to a composition for controlling proliferation of Fusicatenibacter bacteria typified by Fusicatenibacter, the composition containing, as an active ingredient, kojibiose and an oligosaccharide containing kojibiose as a constituent saccharide.

### [Active ingredient]

The composition of the present embodiment contains, as an active ingredient, any one selected from kojibiose and an oligosaccharide containing kojibiose as a constituent saccharide. The oligosaccharide containing kojibiose as a constituent saccharide does not include kojibiose itself. Note that, in the present invention, unless otherwise specified, "any one" is used in the meaning of "at least one". For example, the phrase "any one selected from the group consisting of kojibiose and an oligosaccharide containing kojibiose as a constituent saccharide" includes a case of only kojibiose, a case of only one type of oligosaccharide containing kojibiose as a constituent saccharide, a case of kojibiose and one type of oligosaccharide containing kojibiose as a constituent saccharide, a case of two types of oligosaccharides containing kojibiose as a constituent saccharide, and the like. The phrase "containing as an active ingredient" means that, in the composition, the active ingredient is used in an effective amount for exhibiting an intended function, the active ingredient is used by being specified as a component contributing to a purpose by indication, and the like. The active ingredient may be referred to as a functionally related component (i.e., a component contributing to a specific health purpose (excluding a component related to reduction in disease risk)) in a food with functional claims.

The kojibiose and the oligosaccharide containing kojibiose as a constituent saccharide, used in the composition are not particularly limited as long as they have a desired effect. As for the kojibiose and the oligosaccharide containing kojibiose as a constituent saccharide, used in the composition, one type or a combination of two or more types may be used.

Examples of the oligosaccharide containing kojibiose as a constituent saccharide include galactosyl kojibiose, kojitriose, ceraginose (kojibiosyl glucoside), sentose, and kojibiosyl fructoside, which are trisaccharides, a tetrasaccharide in which one molecule of a monosaccharide such as glucose is bonded to each of these trisaccharides, kojitetraose, and kojitriosyl glucoside.

In a preferable aspect, the composition contains any one of kojibiose and galactosyl kojibiose. Examples thereof include a composition containing kojibiose, a composition containing galactosyl kojibiose, a composition containing kojibiose and galactosyl kojibiose, and a composition containing kojibiose, galactosyl kojibiose, and a tetrasaccharide in which one molecule of glucose is bonded to galactosyl kojibiose.

In the present invention, unless otherwise specified, galactosyl kojibiose refers to one in which galactose is α- or β-bonded to any one of the following positions 1 to 8 of kojibiose. In theory, galactose can be bonded to OH at position 8 in four ways of α-α, α-β, β-α, and β-β, and galactose can be bonded to OH at each of positions 2 to 7 in two ways, and therefore 18 types of galactosyl kojibiose are assumed. These are referred to as 8α-α kojibiose, 8α-β kojibiose, 8β-α kojibiose, 8β-β kojibiose, 1α kojibiose, 2β kojibiose, 2α kojibiose, 2β kojibiose, 3α kojibiose, 3β kojibiose, 4α kojibiose, 4β kojibiose, 5α kojibiose, 5β kojibiose, 6α kojibiose, 6β kojibiose, 7α kojibiose, and 7β kojibiose.

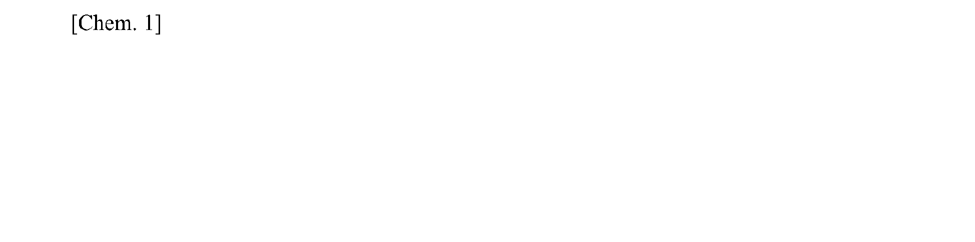

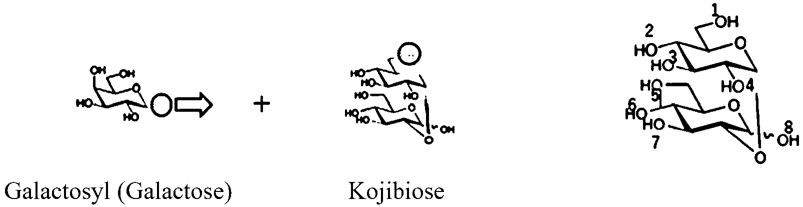

In an aspect, the composition contains any one of the 18 types of galactosyl kojibiose, that is, any one of 8α-α kojibiose, 8α-β kojibiose, 8β-α kojibiose, 8β-β kojibiose, 1α kojibiose, 2β kojibiose, 2α kojibiose, 2β kojibiose, 3α kojibiose, 3β kojibiose, 4α kojibiose, 4β kojibiose, 5α kojibiose, 5β kojibiose, 6α kojibiose, 6β kojibiose, 7α kojibiose, and 7β kojibiose, and may contain 2, 3, 4, 5, 6, 7, 8, 9, 10, 11, 12, 13, 14, 15, 16, and 17, or 18 types of galactosyl kojibiose.

In a preferable aspect, galactosyl kojibiose contained in the composition is α-D-glucopyranosyl-(1→2)-[β-D-galactopyranosyl-(1→4)-] D-glucopyranoside). This saccharide can also be expressed as a type of glucosyllactose.

According to the study of the present inventors, an excellent effect of controlling proliferation of Fusicatenibacter bacteria as one type of Fusicatenibacter bacteria has been confirmed in galactosyl kojibiose. Here, galactosyl kojibiose is a type of oligosaccharide containing kojibiose as a constituent saccharide, and can be degraded by lactase in a gastrointestinal tract of a subject after being administered to the subject to generate kojibiose. Therefore, a saccharide containing kojibiose as a constituent saccharide can generate kojibiose in the intestine in a similar manner, and can exhibit a similar effect to that of galactosyl kojibiose.

The kojibiose and the oligosaccharide containing kojibiose as a constituent saccharide used in the present invention are not particularly limited in terms of their origins and manufacturing methods thereof, and may be manufactured by any method such as a fermentation method, an enzyme method, or an organic synthesis method. One of preferable manufacturing methods is a manufacturing method including a step of allowing glucansucrase or lactic acid bacteria expressing glucansucrase or a treated product thereof to act on a raw material composition containing lactose and sucrose to obtain a composition containing galactosyl kojibiose. In the present invention, unless otherwise specified, glucansucrase refers to an enzyme belonging to glycoside hydrolase (also referred to as glycosyl hydrolases) family 70 (GH70) (See (CAZy) databases and Cantarel et al., Nucleic Acids Res. 37:D233-238, 2009).

When lactic acid bacteria are used, the lactic acid bacteria are preferably lactic acid bacteria expressing glucansucrase. The lactic acid bacteria are preferably, for example, lactic acid bacteria belonging to the Leuconostoc family, more preferably lactic acid bacteria belonging to the genus Leuconostoc, and still more preferably Leuconostoc mesenteroides.

When lactic acid bacteria are used, the lactic acid bacteria are preferably lactic acid bacteria constantly expressing glucansucrase. The lactic acid bacteria are preferably, for example, lactic acid bacteria belonging to the Lactobacillaceae family, more preferably lactic acid bacteria belonging to the genus Liquorilactobacillus or lactic acid bacteria belonging to the genus Limosilactobacillus, and still more preferably Limosilactobacillus reuteri Liquorilactobacillus satsumensis.

When lactic acid bacteria are used, the lactic acid bacteria are preferably lactic acid bacteria constantly expressing glucansucrase, and the glucansucrase is more preferably a bacterial cell binding type. The lactic acid bacteria are preferably, for example, lactic acid bacteria belonging to the Lactobacillaceae family, more preferably lactic acid bacteria belonging to the genus Liquorilactobacillus, and still more preferably Liquorilactobacillus satsumensis. An example of a particularly preferable strain of Liquorilactobacillus satsumensis is JCM12392. Note that JCM12392 is expressed as Lactobacillus satsumensis in RIKEN BioResource Center, GENERAL CATALOG No.9, 2012, JAPAN COLLECTION OF MICROORGANISMS, M51.

Note that when lactic acid bacteria are described in the present invention, unless otherwise specified, the description is in accordance with the reclassified classification according to Zheng J, Wittouck S, Salvetti E, Franz CMAP, Harris HMB, Mattarelli P, O'Toole PW, Pot B, Vandamme P, Walter J, Watanabe K, Wuyts S, Felis GE, Ganzle MG, Lebeer S.:A taxonomic note on the genus Lactobacillus:Description of 23 novel genera, emended description of the genus Lactobacillus Beijerinck 1901, and union of Lactobacillaceae and Leuconostocaceae. Int J Syst Evol Microbiol. 2020 Apr; 70(4):2782-2858. Liquorilactobacillus satsumensis is classified as Lactobacillus satsumensis in accordance with classification before rearrangement.

The lactic acid bacteria expressing glucansucrase or a treated product thereof is preferably any one selected from the group consisting of viable cells, dead cells, a culture containing bacterial cells, disrupted bacterial cells, and a purified product of glucansucrase.

Glucansucrase or the lactic acid bacteria expressing glucansucrase or a treated product thereof, used for manufacturing kojibiose and an oligosaccharide containing kojibiose as a constituent saccharide, can be manufactured using a transformation, chemical synthesis, or a genome editing technique.

A composition containing galactosyl kojibiose, obtained by such a manufacturing method can be used as it is as a solution containing kojibiose and an oligosaccharide containing kojibiose as a constituent saccharide, or after being purified with an ion exchange resin or the like. That is, the composition of the present invention may be a composition containing galactosyl kojibiose, obtained by allowing glucansucrase or lactic acid bacteria expressing glucansucrase or a treated product thereof to act on a composition containing lactose and sucrose.

### [Application]

### (Function, action, and effect)

The composition of the present embodiment can be used to control proliferation of bacteria belonging to the genus Fusicatenibacter (also simply referred to as Fusicatenibacter bacteria). In a preferable aspect, the composition can be used to control proliferation of Fusicatenibacter bacteria in the human intestine. Controlling proliferation of Fusicatenibacter bacteria in the human intestine refers to controlling proliferation of Fusicatenibacter bacteria in a state where various bacteria living in the human intestine are present (also referred to as intestinal bacterial flora). In the present invention, the term "intestinal bacterial flora" refers to bacterial flora in which various bacteria living in the intestine are present, and includes, for example, bacterial flora contained in feces, and is not limited to bacterial flora in the intestine. In the present invention, control includes controlling upward (also referred to as promoting or increasing) and controlling downward (also referred to as suppressing or decreasing), and is preferably controlling upward. Control of proliferation can be control of the number of bacteria or control of an abundance described later.

In the present invention, the intestine refers to a digestive organ in which bacteria are present constantly and which digests and absorbs ingested food in humans and animals. The intestine includes the small intestine and the large intestine, and is preferably the large intestine.

In a preferable aspect, the composition is used to control proliferation of bacteria belonging to the genus Fusicatenibacter in the intestine.

The Fusicatenibacter bacteria include Fusicatenibacter faecihominis and Fusicatenibacter saccharivorans.

In the present invention, the Fusicatenibacter bacteria refer to bacteria identified as the genus Fusicatenibacter by molecular phylogenetic analysis based on the 16S rRNA gene. Determination criteria for a genus by molecular phylogenetic analysis based on the 16S rRNA gene are well known to a person skilled in the art (Stackebrandt E, Ebers J. Taxonomic parameters revisited: tarnished gold standards. Microbiol Today 2006;33:152-155.).

In the present invention, controlling proliferation of specific bacteria in bacterial flora refers to controlling a ratio (abundance) of specific bacteria in the bacterial flora.

Whether or not a certain component controls proliferation of Fusicatenibacter bacteria in the intestine can be evaluated as follows. Feces containing Fusicatenibacter bacteria and provided from a healthy person are added to an appropriate medium and cultured for a certain period as necessary, then a component to be evaluated is added thereto, and the mixture is cultured under appropriate conditions (for example, cultured at 37°C under anaerobic conditions for 48 hours, similar to conditions in the intestine). Then, the number of Fusicatenibacter bacteria in the bacterial flora in the culture is measured. Then, determination can be made by comparing the measurement result with a measurement result for a culture cultured under the same conditions except that a control (for example, sterile water) was added instead of the component to be evaluated.

The number of Fusicatenibacter bacteria contained in the bacterial flora can be determined by a known method. One preferable method is to perform 16S metagenomic analysis (sequence analysis of 16S rRNA gene amplicon) on DNA extracted from the culture. When 16S metagenomic analysis is performed, DNA can be extracted using a commercially available kit. A genome region to be analyzed is not particularly limited as long as bacteria can be determined, and a V3-V4 region of the 16S rRNA gene can be used. As primers, amplification conditions, and a method such as purification of amplicons, for analyzing bacteria, methods well known to a person skilled in the art can be used. Sequence decoding is preferably performed by a next-generation sequencer with higher performance. A next-generation microbiome bioinformatics platform such as QIIME 2^{™} can be used to analyze obtained data. For 16S metagenomic analysis, a person skilled in the art can refer to information such as Sanschagrin S, Yergeau E. Next-generation sequencing of 16S ribosomal RNA gene amplicons. J Vis Exp. 2014;(90):51709. Published 2014 Aug 29. doi:10.3791/51709.

In an aspect, the composition can be used for treatment of any one selected from inflammatory bowel disease and Parkinson's disease. The treatment is not limited to those performed via control of proliferation of Fusicatenibacter bacteria in the intestine unless otherwise specified.

Fusicatenibacter bacteria have been discovered from intestinal bacterial flora of humans. It has been reported that the number of Fusicatenibacter saccharivorans is significantly smaller in a patient suffering from ulcerative colitis than that in a healthy person, and it is considered that inflammation is suppressed by administration of heat-killed bacterial bodies of Fusicatenibacter saccharivorans to oxazolone-induced colitis model mice, and this mechanism is performed via suppression of IL-10 and IL-13 whose production is increased by oxazolone (Non Patent Document 1). In addition, symptom progression of Parkinson's disease is predicted in a patient group having a small number of bacteria belonging to the genus Fusicatenibacter (Non Patent Document 2). Furthermore, it has been reported that Fusicatenibacter bacteria are distributed with high frequency and number of bacteria in an adult, whereas a distribution frequency is low in an elderly person, and the number of bacteria in an elderly person is also small (Non Patent Document 3). Therefore, in an aspect, the composition can be used for treatment of any one selected from inflammatory bowel disease and Parkinson's disease via control of proliferation of Fusicatenibacter bacteria in the intestine.

The inflammatory bowel disease includes ulcerative colitis and Crohn's disease. Parkinson's disease includes juvenile Parkinson's disease that develops at age 40 or less. Treatment of Parkinson's disease includes treatment of any one of symptoms of Parkinson's disease. The symptoms of Parkinson's disease include motor symptoms such as tremor, bradykinesia, muscle rigidity (muscle rigidity), and postural retention disorder, and non-motor symptoms such as constipation, frequent urination, sweating, fatigability, decreased olfaction, orthostatic hypotension, gloomy mood (depression), and diminished interest or motivation (apathy).

When used for treatment of inflammatory bowel disease, the composition can be configured to be free of any of the following:
an oligosaccharide (for example, raffinose, stachiose, galacto-oligosaccharide, isomalto-oligosaccharide, lactosucrose, lactulose, xylo-oligosaccharide, agarooligosaccharide, mannooligosaccharide, or fructo-oligosaccharide), inulin, pectin, pectin processed product, guar gum, guar gum decomposition product, psyllium seed gum, karaya gum, tragacanth gum, gum arabic, indigestible starch, indigestible dextrin, polydextrose, cellulose, hemicellulose, soybean polysaccharide, β-glucan, glucomannan, galactomannan, chondroitin sulfate, hyaluronic acid, levan, lignin, alginic acid and a salt thereof, agarose, and chitosan.

In addition, the composition can be used for treatment of a disease or a condition improved by controlling proliferation of Fusicatenibacter bacteria in the intestine. Such a disease or condition can include various diseases or conditions in addition to inflammatory bowel disease and Parkinson's disease.

In the present invention, treatment of a disease or a condition includes reduction in risk of onset, delay of onset, prevention, medical treatment, and stop and delay of progression. The treatment also includes maintenance of a normal or desired condition, maintenance of ability, and reduction of a symptom (preferably, temporary reduction of a symptom). The treatment further includes assisting improvement from a disease or a condition, alleviating a disease or a condition, and reducing a risk of a disease or a condition. The medical treatment includes curative therapy (medical treatment for removing a cause) and symptomatic therapy (medical treatment for improving a symptom). Actions for improvement or treatment include medical actions performed by a doctor or a nurse, a midwife, or the like who has received instructions from a doctor, and non-therapeutic actions performed by a person other than a doctor, for example, a pharmacist, a dietitian (including national registered dietitian and a sports dietitian), a public health nurse, a midwife, a nurse, a clinical laboratory technician, a sports instructor, a pharmaceutical manufacturer, a pharmaceutical seller, a food manufacturer, a food seller, or the like. Furthermore, the prevention or reduction in risk of onset includes recommendation of ingestion of specific foods and nutrition guidance (including instruction of nutrition necessary for medical care for a sick/injured person and instruction of nutrition for promoting health maintenance).

### (Subject)

A subject of the composition of the present embodiment includes: a subject for whom it is desirable or necessary to control proliferation of Fusicatenibacter bacteria in the intestine; an elderly person; a subject at a risk of any one selected from inflammatory bowel disease and Parkinson's disease; and a subject for whom treatment of any one selected from inflammatory bowel disease and Parkinson's disease is desired or necessary. The elderly person refers to a person who is 65 years old or older.

The subject for whom it is desirable or necessary to control proliferation of Fusicatenibacter bacteria in the intestine includes a subject for whom a relatively low amount of Fusicatenibacter bacteria in the intestine is detected in at least one of the intestine and the feces. The subject for whom control of proliferation of Fusicatenibacter bacteria in the intestine is desired or necessary includes: a subject for whom the number or abundance of Fusicatenibacter bacteria in the intestine is recognized to be relatively small by any examination and analysis such as an intestinal bacterial flora examination, a substance examination in feces, and a fecal metabolome analysis; a subject for whom a risk of any one selected from inflammatory bowel disease and Parkinson's disease is recognized to be high; and a subject to whom ingestion of food and drink or medicine for controlling proliferation of Fusicatenibacter bacteria in the intestine is proposed by a doctor, a nurse, a pharmacist, a dietitian, or the like, or determination of a computer program, based on results of the examination and analysis.

The subject for whom the number or abundance of Fusicatenibacter bacteria in at least one of the intestine and the feces is detected to be relatively small includes, for example, a case where the abundance of the Fusicatenibacter bacteria in a culture solution containing feces collected from the subject is 4% or less, 3% or less, 2% or less, 1.5% or less, 1% or less, or 0.5% or less when the culture solution is cultured at 37°C for 24 hours under anaerobic conditions.

In an aspect, the subject is healthy (not diagnosed as sick by a doctor). The subject may be a human or an animal other than a human.

The age of the subject is not particularly limited, and the subject can be, for example, a neonate (within 28 days after birth); an infant (less than 1 year after birth); a toddler (1 to 6 years after birth); a child (7 years old or older and younger than 15 years old); an adult (15 years old or older); and a person who is 65 years old or older. The composition of the present embodiment is particularly suitable for being administered to an elderly person who is 65 years old or older.

### [Composition]

### (Food composition and the like)

The composition of the present invention can be a food composition or a pharmaceutical composition. The food and the medicine include not only those for humans but also those for animals other than humans, unless otherwise specified. The food includes, unless otherwise specified, a general food, a functional food, a nutritional composition, a therapeutic meal (a meal serving therapeutic purposes, or a meal cooked based on a menu prepared by a dietitian or the like in accordance with a dietary recipe presented by a doctor), a dietary meal, an ingredient adjustment meal, a nursing meal, and a food for medical treatment support. The food includes not only solid products but also liquid products such as a beverage, a health drink, a liquid food, and a soup, unless otherwise specified. The functional food refers to a food that can impart a predetermined functionality to a living body, and includes, for example, general health foods such as a food for specified health uses (including conditional Tokuho [a food for specified health uses]), a food with functional claims, a food with health claims including a food with nutrient function claims, a food for special dietary uses, a nutritional supplement food, a health supplement food, supplement (for example, those of various dosage forms such as tablet, coated tablet, sugar-coated tablet, sugar-coated tablet, enteric coated preparation, capsule (enteric soft capsule, enteric hard capsule, large intestine delivery capsule, or the like), and solution), dietary supplement, food supplement, a medical food (as defined by the Food and Drug Administration (FDA)), and a beauty food (for example, a diet food). In addition, in the present invention, the term "functional food" includes health foods to which a health claim based on the food standard of Codex (Joint FAO/WHO Codex Alimentarius Commission, CAC) is applied. The food supplement is a product that supplements a normal meal and that is obtained by concentrating nutrients or other substances having a nutritional or physiological effect alone or in combination, and a product having a description of Food Supplement. The dietary supplement is a product intended to supplement a meal (excluding tobacco), containing one or more subject components, and having a description of Dietary supplement. The composition may also be a purified product of a particular sugar. The purified product includes a fraction, a partially purified product, and a crude purified product. Note that when indication on a food is actually performed, the indication is performed in accordance with the indication law of each country.

### (Administration route and the like)

The composition of the present invention may be administered orally, parenterally, for example, via a tube (gastric fistula or intestinal fistula), or nasally, and is preferably administered orally. In the present invention, administration is used not only in a case where a medicine is administered to a subject but also in a case where a food or the like other than a medicine is caused to be ingested by a subject. "Administration (administer)" can be read as "ingestion (cause a subject to ingest)", and "administration/ingestion (administer/cause a subject to ingest)" can be read as "administration (administer)".

The composition can be repeatedly administered to a subject and can be continuously administered to a subject for a long period. The period is not particularly limited, and in order to sufficiently exhibit an effect, it is preferable to continuously administer the composition for a relatively long period, for example, 3 days or more, 1 week or more, 2 weeks or more, 1 month or more, 3 months or more, 6 months or more, or 1 year or more.

The composition may be administered on a daily basis, in advance when, for example, risk is high, or when needed. The composition may be administered as a meal, before a meal, after a meal, between meals, or when a disease or a condition desired to be improved by the composition occurs.

### (Dose and content)

In the present embodiment, any one selected from kojibiose as an active ingredient and an oligosaccharide containing kojibiose as a constituent saccharide can be used in an amount acceptable as a food or a medicine. A dosage of the composition of the present embodiment may be any amount as long as an intended effect is exhibited. The dosage can be appropriately set in consideration of various factors such as age, body weight, and symptom of a subject.

A dosage of the composition per day can be an amount acceptable as a food or a medicine, can be 0.5 g or more or 1 g or more, and is preferably 2 g or more, preferably 3 g or more, more preferably 5 g or more, and still more preferably 10 g or more, as an active ingredient amount. An upper limit value of the active ingredient per day can be 80 g or less, 70 g or less, 60 g or less, 50 g or less, 40 g or less, and may be 30 g or less, 20 g or less, or 15 g or less, regardless of a lower limit value. Note that when a plurality of active ingredients are contained in the composition, the amount of the active ingredients refers to the total amount of the contained active ingredients.

Administration may be performed once a day, or may be performed multiple times a day, for example, 2 to 10 times. For example, the dosage of the active ingredient per administration can be 0.5 g or more or 1 g or more, and is preferably 2 g or more, more preferably 3 g or more, and still more preferably 5 g or more. An upper limit value of the active ingredient per administration can be 70 g or less, 60 g or less, 50 g or less, or 40 g or less, and may be 30 g or less, 25 g or less, or 10 g or less, regardless of a lower limit value.

The content of the active ingredient in the composition can be an amount acceptable as a food or a medicine, and can be appropriately set according to a form of the composition. For example, when the composition is in a form of eating or drinking as it is, such as a fermented milk or a beverage, the content of the active ingredient per 100 g of the composition can be 0.01% or more, and is preferably 0.1% or more, more preferably 0.3% or more, and still more preferably 0.5% or more. An upper limit value of the active ingredient per 100 g of the composition can be 8% or less, 5% or less, or 4% or less, and may be 3% or less, regardless of a lower limit value. Alternatively, the content of the active ingredient per solid content of the composition can be 0.1% or more, and is preferably 1% or more, more preferably 3% or more, and still more preferably 5% or more. An upper limit value of the active ingredient per solid content can be 80% or less, 50% or less, or 40% or less, and may be 30% or less, regardless of a lower limit value. Note that in the present invention, % means % by mass unless otherwise specified.

### (Other components and additives)

In the present invention, the composition may contain other active ingredients and nutritional components acceptable as a food or a medicine. Examples of such ingredients/components include lipids (for example, milk fat, vegetable fat and oil, and medium-chain fatty acid-containing fat and oil), proteins (for example, milk protein, milk protein concentrate (MPC), whey protein concentrate (WPC), whey protein isolate (WPI), α-lactalbumin (α-La), β-lactoglobulin (β-Lg), heat-modified whey protein, and enzyme-treated whey protein), amino acids (for example, lysine, arginine, glycine, alanine, glutamic acid, leucine, isoleucine, and valine), carbohydrates other than kojibiose and an oligosaccharide containing kojibiose as a constituent saccharide (glucose, sucrose, fructose, maltose, trehalose, erythritol, maltitol, palatinose, xylitol, and dextrin), electrolytes (for example, sodium, potassium, calcium, and magnesium), vitamins (for example, vitamin A, vitamin B1, vitamin B2, vitamin B6, vitamin B12, vitamin C, vitamin D, vitamin E, vitamin K, biotin, folic acid, pantothenic acid, and nicotinic acids), minerals (for example, copper, zinc, iron, cobalt, and manganese), antibiotics, and dietary fibers.

In the present invention, the composition may contain prebiotics other than the active ingredient. Prebiotics can be referred to as indigestible food components that selectively change proliferation and activity of specific bacteria in the large intestine, thereby advantageously affecting a host and improving health of the host. In the composition, one or more types of prebiotics other than the active ingredient may be used.

The prebiotics other than the active ingredient are not particularly limited as long as the effect of the active ingredient contained in the composition is not impaired. Examples of the prebiotics other than the active ingredient include galactooligosaccharides, fructooligosaccharides (GF2, GF3, and GF4), xylooligosaccharides, isomaltooligosaccharides, raffinose, lactulose, lactosucrose, soybean oligosaccharides, coffee oligosaccharides, dietary fibers, and gluconic acid.

The composition can also be used as a synbiotic, and may contain a probiotic in addition to the active ingredient. The synbiotic is a combination of a probiotic and a prebiotic. The probiotic can be said to be a microorganism that is introduced into the intestine of a host in a living state to produce a beneficial effect for the host.

The probiotics are not particularly limited as long as the effect of the active ingredient contained in the composition is not impaired. Specific lactic acid bacteria and the like are known as examples of the probiotics.

In addition, the composition may further contain an additive acceptable as a food or a medicine. Examples of such an additive include an inert carrier (solid or liquid carrier), an excipient, a surfactant, a binder, a disintegrant, a lubricant, a solubilizing agent, a suspending agent, a coating, a colorant, a preservative, a buffering agent, a pH adjusting agent, an emulsifier, a stabilizer, a sweetener, an antioxidant, a flavor, an acidulant, and a natural product. More specific examples thereof include water, other aqueous solvents, pharmaceutically acceptable organic solvents, collagen, polyvinyl alcohol, polyvinylpyrrolidone, carboxyvinyl polymer, sodium alginate, dextrin, water-soluble dextran, water-soluble dextrin, corn starch, sodium carboxymethyl starch, pectin, xanthan gum, gum arabic, casein, gelatin, agar, glycerin, propylene glycol, polyethylene glycol, petrolatum, paraffin, stearyl alcohol, stearic acid, magnesium stearate, precipitated calcium carbonate, talc, human serum albumin, mannitol, sorbitol, lactose, sucralose, lactose hydrate, maltose syrup powder, white sugar, stevia, aspartame, acesulfame potassium, citric acid, lactic acid, malic acid, tartaric acid, phosphoric acid, acetic acid, fruit juice, and vegetable juice.

### (Dosage form and form)

In an aspect, the food composition may be prepared in any form such as a solid, a liquid, a mixture, a suspension, a powder, a granule, a paste, a jelly, a gel, or a capsule. The food composition according to the present invention can be in any form such as a dairy product, a supplement (for example, a tablet, a coated tablet, a sugar-coated tablet, an enteric coated agent by enteric coating or the like, a capsule (enteric soft capsule, enteric hard capsule, large intestine delivery capsule, or the like), a confection, a beverage, a health drink, a seasoning, a processed food, a daily dish, or a soup. More specifically, the composition of the present invention can be in a form of a liquid meal, a semi-liquid meal, a jelly, a gel, a powder, a modified milk powder, modified liquid milk, a milk powder or liquid milk for pregnant women and lactating women, fermented milk, a bar, a mousse, a chocolate, a biscuit, an ice cream, fermented milk, a lactic acid bacteria beverage, a dairy beverage, a milk beverage, a soft drink, a fruit juice beverage, a tablet, a cheese, bread, a biscuit, a cracker, a pizza crust, a food for sick persons, a nutritional food, a frozen food, a processed food, or the like, and can be in a form of a granule, a powder, a paste, a concentrated liquid, or the like for mixing with a beverage or a food for administration. The granule and the powder can be cubeshaped or stick-shaped (single-dose packaging). In the present invention, the modified milk powder refers to raw milk, cow's milk, special cow's milk, or raw water buffalo milk, or a food manufactured using these as raw materials, processed or used as a main raw material, to which nutrients necessary for infants and toddlers are added to form a powder, as defined in "Ministerial Ordinance Concerning Compositional Standards, etc. for Milk and Milk Products (hereinafter abbreviated as "Ministerial Ordinance Concerning Milk")". In the present invention, the modified liquid milk refers to raw milk, cow's milk, special cow's milk, or raw water buffalo milk, or a food manufactured using these as raw materials, processed or used as a main raw material, to which nutrients necessary for infants and toddlers are added to form a liquid, as defined in Ministerial Ordinance Concerning Milk.

In an aspect, the pharmaceutical composition can also be in any dosage form suitable for oral administration, such as solid preparations such as tablets, granules, powders, pills, and capsules; liquid preparations such as solutions, suspensions, and syrups; gels; and aerosols.

### (Manufacturing method and the like)

In an aspect, in manufacture of the composition, a stage of blending the active ingredient can be appropriately selected. The stage of blending is not particularly limited as long as the properties of the active ingredient are not significantly impaired. For example, the active ingredient can be mixed and blended with raw materials. Alternatively, the active ingredient can be added at a final manufacturing stage to manufacture a composition containing the active ingredient.

### (Indication)

In an aspect, the composition can indicate a purpose of use (application), and in an aspect, the composition indicates a function of the composition or the active ingredient, or a use method thereof based on the function. Examples of the use method to be indicated based on the function are as described above for the function, action, and effect. The composition of the present invention can also indicate that the composition can be used as a prebiotic and can be used as a synbiotic (a combination of a probiotic and a prebiotic). Note that a period such as "temporary" or "long-term" may be appropriately indicated at a beginning of each word.

In an aspect, the composition indicates that administration thereof to a specific subject is recommended. Examples of the subject to be indicated are as described above for the subject.

The indication can be explicit or implicit. Examples of the explicit indication include direct descriptions on tangible objects such as a product itself, a package, a container, a label, and a tag. Examples of the implicit indication (it can also be referred to as implication) include advertisement or publicity on such places or by such means as website, storefront, pamphlet, exhibition, seminar such as media seminar, book, newspaper, magazine, television, radio, mail, e-mail, and voice.

In an aspect, recommendation to ingest the composition is indicated on a personal basis. Such indication can be performed using a document to a subject (whether in writing or electronic), a tablet terminal, a smartphone, and a personal computer of a subject, an SNS of a subject, or the like. In addition, such indication can be performed together with indication of results of any examination and analysis such as an intestinal bacterial flora examination, a substance examination in feces, and a fecal metabolome analysis for a subject. Such indication can also be utilized for precision nutrition (individualized nutrition; it refers to suggesting an appropriate meal (nutrition) according to the body constitution of an individual).

### [Providing apparatus, providing method, and the like]

In the present embodiment, a food information providing apparatus is provided, including:
an information acquisition unit that acquires information on intestinal bacterial flora of a subject;
a deriving unit that derives information on a food to be provided to the subject based on the information on the intestinal bacterial flora; and
a providing unit that provides the derived food information to the subject.

In a preferable aspect, the information acquisition unit of the apparatus acquires information on the presence or absence or the amount of Fusicatenibacter bacteria from the information on the intestinal bacterial flora, and the deriving unit derives information on a food which is a composition containing any one of kojibiose and an oligosaccharide containing kojibiose as a constituent saccharide based on the information on the presence or absence or the amount of Fusicatenibacter bacteria.

In an aspect, any of the following information on a subject can be acquired along with acquisition of the information on intestinal bacterial flora of the subject.
· attribute information of the subject, including any one selected from the group consisting of gender and age;
· test result information regarding a physical/health condition of the subject, including any one selected from the group consisting of height, weight, body mass index (BMI), degree of obesity, body fat, abdominal circumference, blood pressure, lipid, liver/pancreas function, metabolic system, blood, urine, renal function, large intestine, and the like;
· preference/lifestyle survey result information of the subject, including any one selected from the group consisting of eating habit (preference), drinking habit, smoking habit, smoking history, exercise habit, and sleeping time;
· a subjective symptom, stress, a disease currently being treated/under followup, a medicine/health food currently being ingested, medical history, pregnancy/delivery experience, of the subject; and
· business history of the subject, general life of the subject, and others

The acquisition of the information may be based on an answer (for example, an answer to a questionnaire) obtained from the subject in response to a predetermined question.

In an aspect, the food information providing apparatus can indicate food information to be provided on a terminal of the subject. In an aspect, the apparatus further includes an indication unit that indicates food information to be provided. The indication unit can be a terminal of the subject, for example, a tablet terminal, a smartphone, or a personal computer.

In an aspect, the food information providing apparatus may include an analysis unit that analyzes a sample acquired from a subject for bacterial flora, and may further include a receiving unit that receives an order for a food from the subject based on provided food information.

In the present embodiment, a food information providing method is provided, including:
a step of acquiring information on intestinal bacterial flora of a subject;
a step of deriving information on a food to be provided to the subject based on the information on the intestinal bacterial flora; and
a step of providing the derived food information to the subject.

In a preferable aspect, in the step of acquiring information, information on the presence or absence or the amount of Fusicatenibacter bacteria is acquired from the information on the intestinal bacterial flora, and in the step of deriving food information, information on a food which is a composition containing any one selected from kojibiose and an oligosaccharide containing kojibiose as a constituent saccharide is derived based on the information on the presence or absence or the amount of Fusicatenibacter bacteria.

Such a method may further include a step of indicating the food information to be provided on a terminal of the subject. The terminal of the subject can be, for example, a tablet terminal, a smartphone, or a personal computer.

The present embodiment also provides a method for controlling Fusicatenibacter bacteria in the intestine of a subject, or a method for supporting a dietary life or health of a subject, which implements the food information providing method described above, and further implements the following step:
a method for controlling Fusicatenibacter bacteria in the intestine of a subject or a method for supporting a dietary life or health of a subject, including a step of providing a food to the subject based on the derived food information.

The food in the present embodiment is preferably for use as a prebiotic or a synbiotic. The food referred to herein can also be referred to as a food composition, and the description in the above composition section applies as it is.

### [Culturing method and the like]

As another embodiment, there is provided a method for culturing Fusicatenibacter bacteria using any one selected from kojibiose and an oligosaccharide containing kojibiose as a constituent saccharide, or use of any one selected from kojibiose and an oligosaccharide containing kojibiose as a constituent saccharide in culture of Fusicatenibacter bacteria. The culture or use may be performed in intestinal bacterial flora. As another embodiment, there is provided a method for controlling proliferation of Fusicatenibacter bacteria in intestinal bacterial flora, the method including a step of causing any one selected from kojibiose and an oligosaccharide containing kojibiose as a constituent saccharide to be contained in the intestinal bacterial flora containing Fusicatenibacter bacteria. The step of causing the target component to be contained can be achieved by, for example, adding, providing, strengthening, or supplementing the target component.

Hereinafter, the present invention will be described more specifically with reference to Examples. Note that the technical scope of the present invention is not limited to these Examples.

### EXAMPLES

### [Culture Example 1]

### (Feces culture)

Galactosyl kojibiose ((α-D-Glcp-(1→2)-[β-D-Galp-(1→4)-]D-Glcp) was added to a feces diluted solution obtained by suspending feces derived from a healthy adult in a GAM glycolysis semi-fluid medium "NISSUI" (Nissui Pharmaceutical Co., Ltd.) from which agar had been removed by filtration so as to have a concentration of 0.25% (n = 60).

The feces for 60 people were dispensed under refrigerated conditions immediately after excretion, and frozen and stored under a condition of -80°C until use. The feces samples were used and tested for each of the samples without being mixed. Note that the medium used in the test was used after being left to stand at least overnight in an anaerobic glove box. A 96well Deepwell plate was used for culture, and culture was performed at 37°C in an anaerobic environment for one day. Bacterial flora after culture was compared with that under a control condition in which distilled water was added instead of adding galactosyl kojibiose, thereby evaluating a prebiotic ability of the added evaluation substance.

### (Sample)

As the galactosyl kojibiose (α-D-Glcp-(1→2)-[β-D-Galp-(1→4)-]D-Glcp), a trisaccharide fraction mainly containing the galactosyl kojibiose prepared in Manufacturing Example 1 was used.

### (Abundance analysis of intestinal bacteria by next generation sequencer)

DNA was extracted and purified from the fecal diluted solution after culture using Maxwell RSC PureFood GMO & Authentication Kit (Promega), and amplicon sequence of a V3-V4 region by MiSeq (Illumina) was performed. The obtained.fastq file was analyzed by QIIME2 (https://qiime2.org/), and an abundance of each type of intestinal bacteria was calculated.

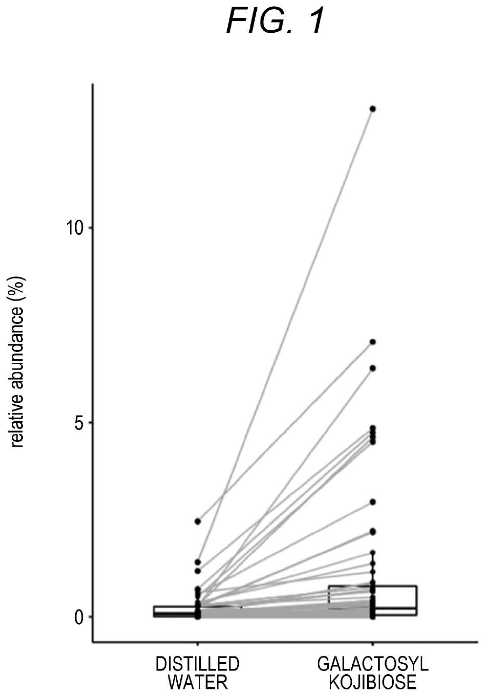

A significant increase in abundance of Fusicatenibacter bacteria was observed when galactosyl kojibiose was added (Fig. 1, Wilcoxon signed rank test, p = 3.63 x 10⁻⁹).

### [Manufacturing Example 1]

### (Preparation of enzyme solution)

Lactobacillus satsumensis JCM12392 was cultured at 30°C overnight using a commercially available MRS liquid medium, and then cooled to 10°C or lower to complete the culture. The JCM12392 strain is available from Japan Collection of Microorganisms (RIKEN BRC-JCM, Japan) of RIKEN BioResource Center (RIKEN BRC) under JCM number JCM12392.

The obtained culture solution was centrifuged and the supernatant was discarded to collect bacteria, and then an equal amount of a citric acid phosphate buffer (pH 5.0) was added thereto, and the bacteria were collected again, thereby washing the bacterial cells. The buffer solution in a half amount of the original culture solution was added to the obtained bacterial cells, and the resulting suspension was used as an enzyme solution.

### (Enzymatic reaction)

32 w/w% of sucrose, 16 w/w% of lactose, and 10 w/w% of the enzyme solution were mixed, and the mixture was caused to react at 48°C for 18 hours under standing conditions. A composition contained 25 to 30% galactosyl kojibiose, 15 to 20% lactose, and 30 to 35% fructose per solid content.

### (Purification of galactosyl kojibiose)

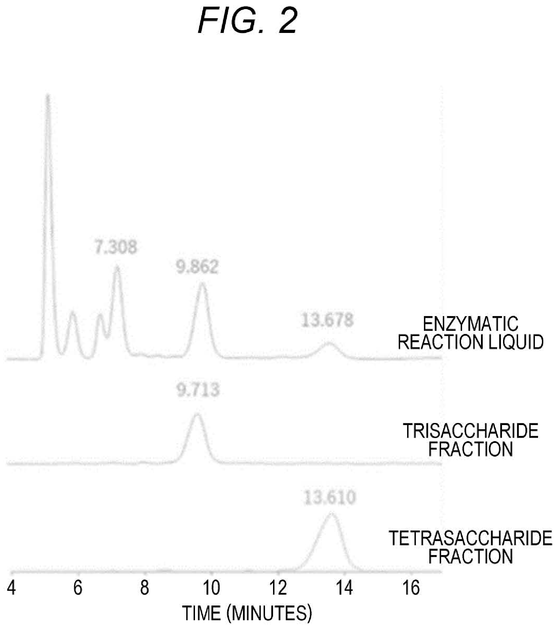

The enzymatic reaction liquid was caused to pass through a carbon celite column, and then distilled water was caused to pass through the column to remove a non-adsorbed substance. Elution was performed stepwise using ethanol aqueous solutions with different concentrations to obtain a trisaccharide fraction mainly containing galactosyl kojibiose (Fig. 2).

### [Summary]

From the above, it can be said that galactosyl kojibiose can be used to control proliferation of Fusicatenibacter bacteria. In addition, galactosyl kojibiose may produce kojibiose by lactase in the intestine. Therefore, it can be said that kojibiose and an oligosaccharide containing kojibiose as a constituent saccharide can be used for control of proliferation of Fusicatenibacter bacteria, and for treatment of a disease or a condition improved by control of proliferation of Fusicatenibacter bacteria.

Fusicatenibacter bacteria have been discovered from intestinal bacterial flora of humans. It has been reported that the number of Fusicatenibacter saccharivorans is significantly smaller in a patient suffering from ulcerative colitis than that in a healthy person, and it is considered that inflammation is suppressed by administration of heat-killed bacterial bodies of Fusicatenibacter saccharivorans to oxazolone-induced colitis model mice, and this mechanism is performed via suppression of IL-10 and IL-13 whose production is increased by oxazolone (Non Patent Document 1). In addition, symptom progression of Parkinson's disease is predicted in a patient group having a small number of bacteria belonging to the genus Fusicatenibacter (Non Patent Document 2). Furthermore, it has been reported that Fusicatenibacter bacteria are distributed with high frequency and number of bacteria in an adult, whereas a distribution frequency is low in an elderly person, and the number of bacteria in an elderly person is also small (Non Patent Document 3). Therefore, it can be said that kojibiose and an oligosaccharide containing kojibiose as a constituent saccharide can be used for treatment of these diseases or conditions. In addition, it can be said that ingestion of a composition containing kojibiose and an oligosaccharide containing kojibiose as a constituent saccharide as a food is useful for supporting a dietary life of a subject and promoting health of the subject. The composition is particularly suitable for being administered to an elderly person who is 65 years old or older as a subject.

### [Food Manufacturing Example 1: Orange 100% fruit juice beverage]

In deionized water, 168 g of 1/6 concentrated orange juice, 20 g of the oligosaccharide composition (solid content: 60%) obtained in Manufacturing Example 1, and an appropriate amount of flavor are dissolved such that the total amount is 1000 ml. This solution is filled in a container, and heat-sterilized at 65°C for 10 minutes to obtain an orange 100% fruit juice beverage containing a trisaccharide fraction mainly containing galactosyl kojibiose. The present fruit juice beverage contains about 0.36 to 0.42 g/100 g of galactosyl kojibiose. This fruit juice beverage can be used for control of proliferation of Fusicatenibacter bacteria.

### [Food Manufacturing Example 2: Drink yoghurt]

A solution obtained by mixing and stirring, under cooling, a solution obtained by mixing and dissolving a raw material cream, a skimmed concentrated milk, and raw material water, heat-sterilizing the mixture at 110°C for 30 seconds, cooling the mixture to 43°C, then adding a lactic acid bacterium starter composed of Lactobacillus bulgaricus and Streptococcus thermophilus to the mixture, fermenting the mixture to acidity of 0.75, and homogenizing the mixture, and a solution obtained by mixing and dissolving raw material water, pectin, sucralose, a trisaccharide fraction mainly containing galactosyl kojibiose, and a flavor, and heat-sterilizing the mixture at 80°C for 10 minutes, is used. Blending ratios of the raw materials are presented in the following Table. The obtained drink yoghurt can be used for treatment of lipid metabolism disorder.

**[Table 1]**

| Raw material | Blending ratio (%) |
|---|---|
| Raw material cream | 1.008 |
| Skimmed milk powder | 22.01 |
| Lactic acid bacterium starter | 1.2 |
| Oligosaccharide composition | 5.5 |
| Sucralose | 0.0064 |
| Pectin | 0.25 |
| Flavor | 0.03 |
| Raw material water | 70.0 |
| Total | 100 |

### [Manufacturing Example 3: Fermented milk 1]

Raw material milk is prepared by mixing 500.0 g of raw milk, 53.2 g of skimmed milk powder, 23.0 g of fresh cream, 403.6 g of tap water, and 50 g of sucrose. The raw material milk is heat-sterilized at a temperature of 95°C, and the heat-sterilized raw material milk is cooled. Thereafter, 0.5% of a Liquorilactobacillus satsumensis concentrated liquid is inoculated into the raw material milk, and the milk is fermented at 48°C for 18 hours under standing conditions, and then Lactobacillus bulgaricus and Streptococcus thermophilus are added thereto as a lactic acid bacterium starter. The added amount of the lactic acid bacterium starter is 20 g. The raw material milk to which the lactic acid bacterium starter has been added is filled into a cup container (volume: 100 ml, made of plastic). The raw material milk filled in the cup container is subjected to stationary fermentation in a fermentation chamber at a temperature of 43°C until acidity of lactic acid reaches 0.7%. The obtained fermented milk can be used for treatment of lipid metabolism disorder.

### [Manufacturing Example 4: Fermented milk 2]

Raw material milk is prepared by mixing 500.0 g of raw milk, 53.2 g of skimmed milk powder, 23.0 g of fresh cream, 403.6 g of tap water, and 54 g of a trisaccharide fraction mainly containing galactosyl kojibiose. Thereafter, the raw material milk is heat-sterilized at a temperature of 95°C, and the heat-sterilized raw material milk is cooled to a temperature of 43°C. Next, Lactobacillus bulgaricus and Streptococcus thermophilus are added to the heat-sterilized raw material milk as a lactic acid bacterium starter. The added amount of the lactic acid bacterium starter is 20 g. The raw material milk to which the lactic acid bacterium starter has been added is filled into a cup container (volume: 100 ml, made of plastic). The raw material milk filled in the cup container is subjected to stationary fermentation in a fermentation chamber at a temperature of 43°C until acidity of lactic acid reaches 0.7%. The obtained fermented milk can be used for treatment of diabetes.

### Industrial Applicability

The present invention supports maintaining and improving health of people by a composition for controlling proliferation of Fusicatenibacter bacteria, the composition containing any one selected from the group consisting of kojibiose and an oligosaccharide containing kojibiose as a constituent saccharide. In addition, according to the present invention, it is possible to provide a food composition that supports maintaining and improving health of people and a method for manufacturing a food. Furthermore, the present invention can improve nutrition of various people, ensure healthy lives of various people, and promote welfare of various people.

## Claims

1. A composition for controlling proliferation of Fusicatenibacter bacteria, comprising any one selected from kojibiose and an oligosaccharide containing kojibiose as a constituent saccharide.

2. The composition according to claim 1, which is for use as a prebiotic or a synbiotic.

3. A composition for treatment of any one selected from inflammatory bowel disease and Parkinson's disease, the composition comprising any one selected from kojibiose and an oligosaccharide containing kojibiose as a constituent saccharide.

4. The composition according to claim 3, wherein the treatment is performed via control of proliferation of Fusicatenibacter bacteria in an intestine.

5. A composition for treatment of a disease or a condition improved by controlling proliferation of Fusicatenibacter bacteria in an intestine, the composition comprising kojibiose and an oligosaccharide containing kojibiose as a constituent saccharide.

6. The composition according to any one of claims 1 to 5, wherein the composition is obtained by allowing glucansucrase to act on a raw material composition containing lactose and sucrose.

7. The composition according to any one of claims 1, 2, 4, and 5, wherein the proliferation control is proliferation promotion.

8. The composition according to any one of claims 1 to 5, comprising galactosyl kojibiose as any one selected from kojibiose and an oligosaccharide containing kojibiose as a constituent saccharide.

9. A food information providing method comprising:
a step of acquiring information on intestinal bacterial flora of a subject;
a step of deriving food information to be provided to the subject based on the information on the intestinal bacterial flora; and
a step of providing the derived food information to the subject, wherein
in the step of acquiring information, information on presence or absence or an amount of Fusicatenibacter bacteria is acquired from the information on the intestinal bacterial flora, and in the step of deriving food information, information on a food which is a composition containing any one selected from the group consisting of kojibiose and an oligosaccharide containing kojibiose as a constituent saccharide is derived based on the information on the presence or absence or the amount of Fusicatenibacter bacteria.

10. A method for controlling Fusicatenibacter bacteria in an intestine of a subject or a method for supporting a dietary life or health of the subject, the method comprising:
a step of performing the method according to claim 9; and
a step of providing a food to the subject based on the derived food information.

11. The method according to claim 9 or 10, wherein the food is for use as a prebiotic or a synbiotic.

12. The method according to claim 9, further comprising a step of indicating the food information to be provided on a terminal of the subject.

13. Use of any one selected from kojibiose and an oligosaccharide containing kojibiose as a constituent saccharide, for control of proliferation of Fusicatenibacter bacteria in intestinal bacterial flora.

14. A method for controlling proliferation of Fusicatenibacter bacteria in intestinal bacterial flora, the method comprising a step of causing any one selected from kojibiose and an oligosaccharide containing kojibiose as a constituent saccharide to be contained in the intestinal bacterial flora containing Fusicatenibacter bacteria.
